(19) Europäisches Patentamt
European Patent Office
Office européen des brevets

(11) Publication number : **0 468 682 A1**

(12) **EUROPEAN PATENT APPLICATION**

(21) Application number : **91306445.7**

(22) Date of filing : **16.07.91**

(51) Int. Cl.[5] : **C07C 237/42,** C07D 205/08, C07D 295/18, C07C 327/38, C07C 271/28, C07C 275/42, C07C 323/52, C07C 317/44, A01N 37/46, A01N 53/00, A01N 43/44, // A01N47/20, A01N47/30

(30) Priority : **27.07.90 GB 9016580**

(43) Date of publication of application : **29.01.92 Bulletin 92/05**

(84) Designated Contracting States : **CH DE ES FR GB IT LI NL**

(71) Applicant : **IMPERIAL CHEMICAL INDUSTRIES PLC**
**Imperial Chemical House, Millbank**
**London SW1P 3JF (GB)**

(72) Inventor : **Crowley, Patrick Jelf**
**56 Ellis Avenue**
**Crowthorne, Berkshire (GB)**
Inventor : **Glen, Alasdair Thomas**
**5 Hall Grove**
**Macclesfield, Cheshire SK10 2HQ (GB)**
Inventor : **Spence, Rosamund Alison**
**Chazey Court Farm, The Warren**
**Caversham, Berkshire RG4 7TQ (GB)**
Inventor : **Lawson, Kevin Robert**
**High Stile, Piddington Lane**
**Piddington, High Wycombe, Bucks HP14 3BB (GB)**

(74) Representative : **Houghton, Malcolm John et al**
**Imperial Chemical Industries PLC Legal Department: Patents PO Box 6 Bessemer Road**
**Welwyn Garden City Hertfordshire AL7 1HD (GB)**

(54) **Fungicides.**

(57) Fungicidal compounds of formula (I) :

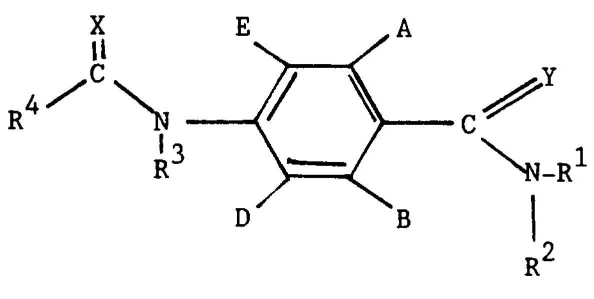

(I)

in which D and E are independently H or fluoro ; X and Y are O or S ; A, B, $R^3$ and $R^4$ have various specified values $R^1$ is $C_{2-4}$ alkenyl (optionally substituted with halogen), $C_{2-4}$ alkynyl (optionally substituted with halogen), halo($C_{1-4}$)alkyl, $C_{3-6}$ cycloalkyl, $C_{3-6}$ cycloalkyl($C_{1-4}$)alkyl, $C_{1-4}$ alkoxy($C_{1-4}$)alkyl, $C_{1-4}$ alkylthio($C_{1-4}$)alkyl or cyano ; and $R^2$ is $C_{1-4}$ alkyl, $C_{1-4}$ alkoxy, $C_{2-4}$ alkenyl (optionally substituted with halogen), $C_{2-4}$ alkynyl (optionally substituted with halogen), halo($C_{1-4}$) alkyl, $C_{3-6}$ cycloalkyl, $C_{3-6}$ cycloalkyl($C_{1-4}$)alkyl, $C_{1-4}$alkoxy($C_{1-4}$)alkyl, $C_{1-4}$ alkylthio($C_{1-4}$)alkyl or cyano.

EP 0 468 682 A1

This invention relates to novel fungicidal acylaminobenzamides, to processes for preparing them, to fungicidal compositions containing them and to methods of using them to combat fungi, especially fungal infections of plants.

Acknowledgement is made of UK Application No. 42454/77 from which US Patent No. 4282218, for example, claims priority and of EP-A-0127990. The former describes acylanilides which have antiandrogenic properties and the latter describes aniline derivatives which have fungicidal properties.

According to the present invention there is provided a compound of the formula (I), in which A and B are independently H, fluoro, chloro, bromo, iodo, $C_{1-4}$ alkyl, $C_{1-4}$ alkoxy, halo($C_{1-4}$)alkyl, cyano, $C_{1-4}$ alkoxycarbonyl, $C_{1-4}$ alkoxymethyl, $C_{1-4}$ alkylthiomethyl, $C_{2-4}$ alkenyl, $C_{2-4}$ alkynyl, formyl, $C_{1-4}$ alkylthio, carboxy, $C_{1-4}$ alkylcarbonyl or nitro, provided that both A and B are not H; D and E are independently H or fluoro; $R^1$ is $C_{2-4}$ alkenyl (optionally substituted with halogen), $C_{2-4}$ alkynyl (optionally substituted with halogen), halo($C_{1-4}$) alkyl, $C_{3-6}$ cycloalkyl, $C_{3-6}$ cycloalkyl($C_{1-4}$)alkyl, $C_{1-4}$ alkoxy($C_{1-4}$)alkyl, $C_{1-4}$ alkylthio($C_{1-4}$)alkyl or cyano; $R^2$ is $C_{1-4}$ alkyl, $C_{1-4}$ alkoxy, $C_{2-4}$ alkenyl (optionally substituted with halogen), $C_{2-4}$ alkynyl (optionally substituted with halogen), halo($C_{1-4}$)alkyl, $C_{3-6}$ cycloalkyl, $C_{3-6}$ cycloalkyl($C_{1-4}$)alkyl, $C_{1-4}$ alkoxy($C_{1-4}$)alkyl, $C_{1-4}$ alkylthio($C_{1-4}$)alkyl or cyano; $R^3$ is H; $R^4$ is alkyl, $C_{2-8}$ alkenyl, $C_{2-8}$ alkynyl or cyclopropyl (all of which are optionally substituted with halogen, $C_{1-4}$ alkyl, $C_{1-4}$ alkoxy or $C_{1-4}$ alkylthio), or $R^3$ and $R^4$, together with the group C(O)N to which they are attached, join to form an azetidin-2-one ring which is optionally substituted with $C_{1-4}$ alkyl; and X and Y are independently oxygen or sulphur.

A preferred value of $R^4$ is $R(CH_3)_2C$- in which R is halogen (especially fluoro), $C_{1-4}$ alkyl (especially methyl or ethyl) or $C_{1-4}$ alkoxy (especially methoxy).

Alkyl groups and the alkyl moiety of other alkyl-containing groups can be in the form of straight or branched chains. Examples are methyl, ethyl, propyl (n-and iso-propyl), butyl (n-, sec, iso- and t-butyl), 1,1-dimethylpropyl and 1,1-dimethylbutyl. Alkenyl and alkynyl groups can also be in the form of straight or branched chains. Examples are 1,1-dimethylbut-3-enyl and 1,1-dimethylprop-2-ynyl.

Halogen includes fluorine, chlorine and bromine.

In one aspect the invention provides a compound of formula (I) in which A is chloro or bromo; B and $R^3$ are both H; X and Y are both 0; $R^1$ is $C_{2-4}$ alkenyl, $C_{2-4}$ alkynyl, halo($C_{1-4}$)alkyl, cyclopropyl, $C_{1-4}$alkoxy($C_{1-4}$)-alkyl, or $C_{1-4}$ alkylthio($C_{1-4}$)alkyl; and $R^2$ is methyl.

In another aspect the invention provides a compound of formula (I) in which A is halo, methyl, $CF_3$, ethenyl or ethynyl; B is H or fluoro; E and D are both H; $R^1$ is allyl, 2-chloro- or 2-bromoalkyl, propargyl, 2-monofluoro- or 2-trifluoroethyl, cyclopropyl, methylthiomethyl, methoxyethyl or cyclopropylmethyl; $R^2$ is methyl, ethyl, allyl or propargyl; $R^3$ is H; and $R^4$ is $(CH_3)_3C$, $F((CH_3)_2C$, $CH_3CH_2(CH_3)_2C$, $F(CH_3CH_2)(CH_3)C$, $F((CH_3)_2C)(CH_3)C$, $F(CH_3CH_2)(CH_3CH_2)C$ or $F(FCH_2)(CH_3)C$.

The invention is illustrated by the compounds listed in Table I which follows. The compounds have the general formula (I.1) in which the values A, B, $R^1$, $R^2$ and $R^4$ are as listed.

TABLE I

| Compound No | $R^1$ | $R^2$ | $R^4$ | A | B | mpt (°C) |
|---|---|---|---|---|---|---|
| 1 | $-CH_2CH{=}CH_2$ | $CH_3$ | $(CH_3)_3C$ | Cl | H | 154-157 |
| 2 | $-CH_2C{\equiv}CH$ | $CH_3$ | $(CH_3)_3C$ | Cl | H | 178-179 |
| 3 | $-CH_2CH_2F$ | $CH_3$ | $(CH_3)_3C$ | Cl | H | 143-146 |
| 4 | $-CH_2CF_3$ | $CH_3$ | $(CH_3)_3C$ | Cl | H | |
| 5 | cyclopropyl | $CH_3$ | $(CH_3)_3C$ | Cl | H | 148-149 |
| 6 | $-CH_2SCH_3$ | $CH_3$ | $(CH_3)_3C$ | Cl | H | 161-163 |
| 7 | $-CH_2CH_2OCH_3$ | $CH_3$ | $(CH_3)_3C$ | Cl | H | |
| 8 | $-CH_2CH{=}CH_2$ | $CH_3$ | $F(CH_3)_2C$ | Cl | H | |
| 9 | $-CH_2C{\equiv}CH$ | $CH_3$ | $F(CH_3)_2C$ | Cl | H | Oil* |
| 10 | $-CH_2CH_2F$ | $CH_3$ | $F(CH_3)_2C$ | Cl | H | |
| 11 | $-CH_2CF_3$ | $CH_3$ | $F(CH_3)_2C$ | Cl | H | |
| 12 | cyclopropyl | $CH_3$ | $F(CH_3)_2C$ | Cl | H | |
| 13 | $-CH_2SCH_3$ | $CH_3$ | $CH_3CH_2(CH_3)_2C$ | Cl | H | |
| 14 | $-CH_2CH_2OCH_3$ | $CH_3$ | $CH_3CH_2(CH_3)_2C$ | Cl | H | |
| 15 | $-CH_2CH{=}CH_2$ | $CH_3$ | $F(CH_3)_2C$ | Br | H | |
| 16 | $-CH_2C{\equiv}CH$ | $CH_3$ | $F(CH_3)_2C$ | Br | H | |

TABLE I (continued)

| Compound No | $R^1$ | $R^2$ | $R^4$ | A | B | mpt (°C) |
|---|---|---|---|---|---|---|
| 17 | $-CH_2CH_2F$ | $CH_3$ | $F(CH_3)_2C$ | Br | H | |
| 18 | cyclopropyl | $CH_3$ | $F(CH_3)_2C$ | Br | H | |
| 19 | $-CH_2SCH_3$ | $CH_3$ | $F(CH_3)_2C$ | Br | H | |
| 20 | $-CH_2CH_2OCH_3$ | $CH_3$ | $F(CH_3)_2C$ | Br | H | |
| 21 | $-CH_2CH=CH_2$ | $CH_3CH_2$ | $F(CH_3)_2C$ | Cl | H | Oil* |
| 22 | $-CH_2C\equiv CH$ | $CH_3CH_2$ | $F(CH_3)_2C$ | Cl | H | |
| 23 | $-CH_2CH=CH_2$ | $CH_3$ | $F(CH_3)_2C$ | F | H | |
| 24 | $-CH_2C\equiv CH$ | $CH_3$ | $F(CH_3)_2C$ | F | H | |
| 25 | $-CH_2CH_2F$ | $CH_3$ | $F(CH_3)_2C$ | F | H | |
| 26 | $-CH_2CH_2F$ | $CH_3CH_2$ | $F(CH_3)_2C$ | F | H | |
| 27 | cyclopropyl | $CH_3$ | $F(CH_3)_2C$ | F | H | |
| 28 | cyclopropyl | $CH_3CH_2$ | $F(CH_3)_2C$ | F | H | |
| 29 | $-CH_2CF_3$ | $CH_3$ | $F(CH_3)_2C$ | F | H | |
| 30 | cyclopropyl | $-CH_2CH=CH_2$ | $F(CH_3)_2C$ | Cl | H | |
| 31 | cyclopropyl | $-CH_2CH=CH_2$ | $F(CH_3)_2C$ | F | H | |
| 32 | cyclopropyl | $-CH_2C\equiv CH$ | $F(CH_3)_2C$ | F | H | |
| 33 | $-CH_2CCl=CH_2$ | $CH_3$ | $F(CH_3)_2C$ | Cl | H | |
| 34 | $-CH_2CBr=CH_2$ | $CH_3$ | $F(CH_3)_2C$ | Cl | H | |
| 35 | $-CH_2CH=CH_2$ | $CH_3$ | $F(CH_3CH_2)(CH_3)C$ | Cl | H | |
| 36 | $-CH_2CH=CH_2$ | $CH_3$ | $F((CH_3)_2C)(CH_3)C$ | Cl | H | |
| 37 | $-CH_2CH=CH_2$ | $CH_3$ | $F((CH_3CH_2)(CH_3CH_2)C$ | Cl | H | |
| 38 | $-CH_2CH=CH_2$ | $CH_3$ | $F(CH_3)_2C$ | F | F | |
| 39 | $-CH_2C\equiv CH$ | $CH_3$ | $F(CH_3)_2C$ | F | F | |
| 40 | cyclopropyl | $CH_3$ | $F(CH_3)_2C$ | F | F | |
| 41 | cyclopropyl | $CH_3$ | $F(CH_3)_2C$ | I | H | |
| 42 | $-CH_2CH=CH_2$ | $CH_3$ | $F(CH_3)_2C$ | I | H | |
| 43 | cyclopropyl | $CH_3$ | $F(CH_3)_2C$ | $CH_2=CH$ | H | |
| 44 | cyclopropyl | $CH_3$ | $F(CH_3)_2C$ | $CH\equiv C$ | H | |
| 45 | cyclopropyl | $CH_3$ | $F(CH_3)_2C$ | $CH_3$ | H | |
| 46 | cyclopropyl | $CH_3$ | $F(CH_3)_2C$ | $CF_3$ | H | |
| 47 | $-CH_2C\equiv CH$ | $CH_3$ | $F(CH_3CH_2)(CH_3)C$ | Cl | H | |

TABLE I (continued)

| Compound No | $R^1$ | $R^2$ | $R^4$ | A | B mpt (°C) |
|---|---|---|---|---|---|
| 48 | cyclopropyl | $CH_3$ | $F(CH_3CH_2)(CH_3)C$ | Cl | H |
| 49 | $-CH_2CH=CH_2$ | $CH_3$ | $F(CH_3CH_2)(CH_3)C$ | F | H |
| 50 | $-CH_2C\equiv CH$ | $CH_3$ | $F(CH_3CH_2)(CH_3)C$ | F | H |
| 51 | cyclopropyl | $CH_3$ | $F(CH_3CH_2)(CH_3)C$ | F | H |
| 52 | $-CH_2CH=CH_2$ | $CH_3$ | $F(CH_3CH_2)(CH_3)C$ | F | F |
| 53 | $-CH_2C\equiv CH$ | $CH_3$ | $F(CH_3CH_2)(CH_3)C$ | F | F |
| 54 | cyclopropyl | $CH_3$ | $F(CH_3CH_2)(CH_3)C$ | F | F |
| 55 | cyclopropylmethyl | $CH_3$ | $F(CH_3)_2C$ | Cl | H |
| 56 | cyclopropylmethyl | $CH_3$ | $F(CH_3)_2C$ | F | H |
| 57 | cyclopropylmethyl | $CH_3$ | $F(CH_3)_2C$ | F | F |
| 58 | $-CH_2CH=CH_2$ | $CH_3$ | $F(FCH_2)(CH_3)C$ | Cl | H |
| 59 | $-CH_2C\equiv CH$ | $CH_3$ | $F(FCH_2)(CH_3)C$ | Cl | H |
| 60 | $-CH_2CH=CH_2$ | $CH_3$ | $F(FCH_2)(CH_3)C$ | F | H |
| 61 | $-CH_2C\equiv CH$ | $CH_3$ | $F(FCH_2)(CH_3)C$ | F | H |

* Compound No. 9 (pair of rotamers) $^1H$ NMR (270MHz, $CDCl_3$) δ 1.64 (6H,s), 1.28-1.34(1H,m), 2.94 and 3.18(3H,each s), 3.90 and 4.40(2H each bd), 7.22-7.31(1H,m), 7.46-7.54(1H,m), 7.84(1H,m), 8.56(1H,bd) ppm.

* Compound No. 21 (pair of rotamers) $^1H$ NMR (270MHz, $CDCl_3$) δ 1.05 and 1.22 (3H,each t), 1.66(6H,d), 3.23 and 3.56(2H,each q), 3.79 and 4.18(2H,each d), 5.10-5.30(2H,m), 5.62-5.96(1H,m), 7.18-7.35(2H,m), 7.61-7.70(1H,m), 8.3(1H,m) ppm.

The compounds of the invention can be made by, for example, the methods illustrated in Schemes 1 and 2. Throughout these Schemes $R^1$, $R^2$, $R^4$, A, B, D, and E are as defined before.

In Scheme 1, compounds of formula (II) can be prepared by reacting compounds of formula (VI) with an acid chloride $R^4COCl$ in a suitable organic solvent such as methylene chloride or toluene in the presence of a base such as a tertiary amine (for example triethylamine) or an alkali metal carbonate or hydroxide (for example sodium bicarbonate or sodium hydroxide).

Compounds of formula (VI) can be made by reduction of nitro compounds of formula (V) using standard methods known in the literature, such as iron powder in aqueous ethanol.

Compounds of formula (V) can be made by treatment of compounds of formula (IV) with a base (for example sodium hydride) and a compound of formula $R^2Z$ (where Z is a leaving group such as chloride, bromide, iodide or tosylate) in a suitable solvent (for example DMF or THF).

Amides of formula (IV) can be made from acid chlorides of formula (III) by reaction with an amine $R^1R^2NH$ in a suitable organic solvent (such as methylene chloride or toluene) or in water, in the presence of a base (such as triethylamine or sodium bicarbonate or excess amine $R^1R^2NH$).

In Scheme 2, compounds of formula (II) can be prepared from compounds of formula (IX) by reaction with an amine $R^1R^2NH$ in a suitable organic solvent such as methylene chloride or tetrahydrofuran (THF) in the presence of a base such as triethylamine, sodium bicarbonate or excess $R^1R^2NH$.

Acid chlorides of formula (IX) can be prepared from carboxylic acids of formula (VIII) by reaction with a standard reagent such as oxalyl chloride in a suitable dry solvent such as THF or methylene chloride and with a catalytic quantity of DMF being added if necessary.

Carboxylic acids of formula (VIII) can be prepared from the appropriately substituted 4-aminobenzoic acid (VII) by reaction with an acid chloride $R^4COCl$ in water in the presence of at least two equivalents of a base such as an alkali metal carbonate or hydroxide (for example sodium bicarbonate). The substituted 4-aminobenzoic acids (VII) can generally be made by methods described in the literature.

The compounds of the invention may also be prepared using methods and techniques described in EP-A-0381330 and in UK Applications Nos. 9016577.0, 9016581.2 and 9016582.0 and applications claiming priority therefrom, the contents of which are incorporated herein by reference.

In another aspect, the invention provides processes as herein described for preparing the compounds of the invention.

The compounds of the invention show fungicidal activity across a range of plant diseases. They are, however, particularly active against the class of pathogens known as the phycomycetes (equivalent to the oomycetes). These include species of Phytophthora, Plasmopara, Peronospora and Pseudoperonospora. Examples of pathogens which the invention compounds are particularly useful for controlling are: Plasmopara viticola on vines; other downy mildews such as Bremia lactucae on lettuce; Peronospora spp. on soybeans, tobacco, onions and other hosts; Pseudoperonospora humuli on hops and Pseudoperonospora cubensis on cucurbits; Phytophthora infestans on potatoes and tomatoes and other Phytophthora spp. on vegetables, strawberries, avocado, pepper, ornamentals, tobacco, cocoa and other hosts; and Pythium sp on rice, horticultural plants, vegetables and turf.

The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems. The invention compounds may also be injected into plants or sprayed onto vegetation using electrodynamic spraying techniques or other low volume methods.

The term "plant" as used herein includes seedlings, bushes and trees. Furthermore, the fungicidal method of the invention includes preventative, protectant, prophylactic and eradicant treatments.

The compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrolidone, propylene glycol or N,N-dimethylformamide). The compositions may also be in the form of wettable powders or water dispersible granules comprising wetting or dispersing agents to facilitate the dispersion in liquids. The powders and granules may also contain fillers and suspending agents.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as cyclohexanone and methylcyclohexanone, chlorinated hydrocarbons such as chlorobenzene and trichlorethane, and alcohols such as benzyl alcohol, furfuryl alcohol,

butanol and glycol ethers.

Suspension concentrates of largely insoluble solids may be prepared by ball or bead milling with a dispersing agent with a suspending agent included to stop the solid settling.

Compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives. For example, certain mineral oil and natural plant oil (such as soya bean and rape seed oil) additives have been found to enhance several-fold foliar protectant activity against, for example, Plasmopara viticola.

The invention compounds can be used as mixtures with fertilisers (e.g. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising a fertiliser and the compound of general formula (I) or a salt or metal complex thereof.

Wettable powders, emulsifiable concentrates and suspension concentrates will normally contain surfactants, e.g. a wetting agent, dispersing agent, emulsifying agent or suspending agent. These agents can be cationic, anionic or non-ionic agents.

Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example, polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient. After dilution to form aqueous preparations, such preparations may contain varying amounts of the active ingredient depending upon the intended purpose, but an aqueous preparation containing 0.0005% or 0.01% to 10% by weight of active ingredient may be used.

The compositions of this invention may contain other compounds having biological activity, e.g. compounds having similar or complementary fungicidal activity or which possess plant growth regulating, herbicidal or insecticidal activity.

A fungicidal compound which may be present in the composition of the invention may be one which is capable of combating ear diseases of cereals (e.g. wheat) such as Septoria, Gibberella and Helminthosporium spp., seed and soil-borne diseases and downy and powdery mildews on grapes and powdery mildew and scab on apple, etc. By including another fungicide, the composition can have a broader spectrum of activity than the compound of general formula (I) alone. Further the other fungicide can have a synergistic effect on the fungicidal activity of the compound of general formula (I). Examples of fungicidal compounds which may be included in the composition of the invention are (RS)-1-aminopropylphosphonic acid, (RS)-4--(4-chlorophenyl)-2-phenyl-2-(1H-1,2,4-triazol-1-ylmethyl)butyronitrile, (Z)-N-but-2-enyloxymethyl-2-chloro-2′,6′-diethylacetanilide, 1-(2-cyano-2-methoxyiminoacetyl)-3-ethyl urea, 3-(2,4-dichlorophenyl)-2-(1H-1,2,4-triazol-1-yl)quinazolin-4(3H)-one, 4-bromo-2-cyano-N,N-dimethyl-6-trifluoromethylbenzimidazole-1-sulphonamide, 5-ethyl-5,8-dihydro-8-oxo(1,3)-dioxol-(4,5-g)quinoline-7-carboxylic acid, α-[N-(3-chloro-2,6-xylyl)-2-methoxyacetamido]-γ-butyrolactone, aldimorph, anilazine, benalaxyl, benomyl, biloxazol, binapacryl, bitertanol, blasticidin S, bromuconazole, bupirimate, buthiobate, captafol, captan, carbendazim, carboxin, chlorbenzthiazone, chloroneb, chlorothalonil, chlorozolinate, copper containing compounds such as copper oxychloride, copper sulphate and Bordeaux mixture, cycloheximide, cymoxanil, cyproconazole, cyprofuram, di-2-pyridyl disulphide 1,1′-dioxide, dichlofluanid, dichlone, diclobutrazol, diclomezine, dicloran, difenoconazole, dimethamorph, dimethirimol, diniconazole, dinocap, ditalimfos, dithianon, dodemorph, dodine, edifenphos, etaconazole, ethirimol, ethyl (Z)-N-benzyl-N-([methyl(methylthioethylideneamino-oxycarbonyl)amino]thio)-β-alaninate, etridiazole, fenapanil, fenarimol, fenfuram, fenpiclonil, fenpropidin, fenpropimorph, fentin acetate, fentin hydroxide, flutolanil, flutriafol, flusilazole, folpet, fosetyl-aluminium, fuberidazole, furalaxyl, furconazole-cis, guazatine, hexaconazole, hydroxyisoxazole, imazalil, imibenconazole, iprobenfos, iprodione, isoprothiolane, kasugamycin, mancozeb, maneb, mepanipyrim, mepronil, metalaxyl, methfuroxam, metsulfovax, myclobutanil, neoasozin, nickel dimethyldithiocarbamate, nitrothal-isopropyl, nuarimol, ofurace, organomercury compounds, oxadixyl, oxycarboxin, pefurazoate, penconazole, pencycuron, phenazin oxide, phthalide, polyoxin D, polyram, probenazole, prochloraz, procymidone, propamocarb, propiconazole, propineb, prothiocarb, pyrazophos, pyrifenox, pyroquilon, pyroxyfur, pyrrolnitrin, quinomethionate, quintozene, SSF-109, streptomycin, sulphur, tebuconazole, techlofthalam, tecnazene, tetraconazole, thiabendazole, thicyofen, thiophanate-methyl, thiram, tolclofos-methyl, triacetate salt of 1,1′-iminodi(octamethylene)diguanidine, triadimefon, triadimenol, triazbutyl, tricyclazole, tridemorph, triforine, validamycin A, vinclozolin, zarilamid and zineb. The compounds of general formula (I) can be mixed with soil, peat or other rooting media for the protection of plants against seed-borne, soil-borne or foliar fungal diseases.

Suitable insecticides which may be incorporated in the composition of the invention include buprofezin, carbaryl, carbofuran, carbosulfan, chlorpyrifos, cycloprothrin, demeton-s-methyl, diazinon, dimethoate, ethofenprox, fenitrothion, fenobucarb, fenthion, formothion, isoprocarb, isoxathion, monocrotophos, phenthoate, pirimicarb, propaphos and XMC.

Plant growth regulating compounds are compounds which control weeds or seedhead, formation, or selectively control the growth of less desirable plants (e.g. grasses).

Examples of suitable plant growth regulating compounds for use with the invention compounds are 3,6-dichloropicolinic acid, 1-(4-chlorophenyl)-4,6-di-methyl-2-oxo-1,2-dihydropyridine-3-carboxylic acid, methyl-3,6-dichloroanisate, abscisic acid, asulam, benzoylprop-ethyl, carbetamide, daminozide, difenzoquat, dikegulac, ethephon, fenpentezol, fluoridamid, glyphosate, glyphosine, hydroxybenzonitriles (e.g. bromoxynil), inabenfide, isopyrimol, long chain fatty alcohols and acids, maleic hydrazide, mefluidide, morphactins (e.g. chlorfluoroecol), paclobutrazol, phenoxyacetic acids (e.g. 2,4-D or MCPA), substituted benzoic acid (e.g. triiodobenzoic acid), substituted quaternary ammonium and phosphonium compounds (e.g. chloromequat, chlorphonium or mepiquatchloride), tecnazene, the auxins (e.g. indoleacetic acid, indolebutyric acid, naphthylacetic acid or naphthoxyacetic acid), the cytokinins (e.g. benzimidazole, benzyladenine, benzylaminopurine, diphenylurea or kinetin), the gibberellins (e.g. $GA_3$, $GA_4$ or $GA_7$) and triapenthenol.

The following Examples illustrate the invention.

Where shown, infrared and NMR data are selective; no attempt is made to list every absorption in all cases. The following abbreviations are used throughout:

| | | | | | |
|---|---|---|---|---|---|
| THF | = | tetrahydrofuran | d | = | doublet |
| DMF | = | N,N-dimethylformamide | dd | = | doublet or doublets |
| NMR | = | nuclear magnetic resonance | t | = | triplet |
| mp | = | melting point | m | = | multiplet |
| s | = | singlet | b | = | broad |

## EXAMPLE 1

This illustrates the preparation of 2-chloro-4-(2′,2′-dimethyl-(propionamido)-N-cyclopropyl-N-methylbenzamide (Compound No. 5 of Table I).

Step 1

Preparation of 2-chloro-4-nitro- N-cyclopropylbenzamide.

2-Chloro-4-nitrobenzoyl chloride (11.69g) in dry THF (50ml) was added dropwise over 0.5 hr to a stirred solution of cyclopropylamine (3.14g) and triethylamine (5.57g) in dry THF (50ml) at 0-5°C.

The mixture was stirred at 0-10°C for 1 hour after completion of the addition, warmed to room temperature and poured into dilute aqueous hydrochloric acid (500ml) and extracted with ethyl acetate. The organic layer was dried over magnesium sulphate, and evaporated to give the product as a pale yellow solid (11.87g). Recrystallisation of a small sample gave material with mp 161-162°C; $^1H$ NMR ($CDCl_3$, 270MHz) δ: 0.68(2H,m), 0.93(2H,m), 2.95(1H,m), 6.23(1H,bs), 7.80(1H,d), 8.17(1H,dd), 8.28(1H,d) ppm.

Step 2

Preparation of 2-chloro-4-nitro-N-cyclopropyl-N-methylbenzamide.

2-Chloro-4-nitro-N- cyclopropylbenzamide (11.0g) from the previous reaction in dry DMF (50ml) was added dropwise at room temperature to a stirred suspension of sodium hydride (2.40g of a 60% dispersion in oil, washed with light petroleum before use) in 50ml. After completion of the addition, the mixture was heated to 50-60°C for 1 hour, and then cooled to room temperature. Neat methyl iodide (8.52g) was then added over 15 minutes, keeping the temperature below 30°C. After stirring for 1 hour, the mixture was poured into water, and was then extracted with ethyl acetate. The organic extract was washed with water, dried over magnesium sulphate and evaporated to give the crude product as an orange-yellow solid (12.09g). Recrystallisation of a small sample from cyclohexane gave material with mp 84-85°C; $^1H$ NMR ($CDCl_3$, 270MHz) δ: 0.54(4H,d), 2.70(1H,quintet), 3.14(3H,s), 7.48(1H,d), 8.18(1H,dd), 8.30(1H,d) ppm.

Step 3

Preparation of 4-amino-2-chloro-N-cyclopropyl-N-methylbenzamide.

2-Chloro-4-nitro-N-cyclopropyl-N-methyl benzamide (11.0g) from the previous reaction, in ethanol (60ml) was added to iron powder (10.0g) in water (15ml) and ethanol (60ml), containing concentrated hydrochloric acid (5ml), and the mixture heated to 50-60°C for 5 hours, and then cooled overnight. The reaction mixture was filtered (celite), evaporated to 50ml, treated with water (100ml) and concentrated hydrochloric acid (10ml), and then washed with ethyl acetate. The aqueous portion was then basified with aqueous bicarbonate and extracted with methylene chloride (150ml) and ethanol (30ml). After filtration through celite, the organic layer was washed with water, dried over magnesium sulphate and evaporated to give the crude product as a greenish solid (8.16gm). Recrystallisation of a small sample from ethyl acetate gave material with mp 123-125°C; $^1H$ NMR ($CDCl_3$, 270 MHz) δ: 0.50(4H,m), 2.78(1H,d), 3.10(3H,s), 3.80(2H,s), 6.55(1H,dd), 7.05(1H,d), 7.24(1H,d) ppm.

Step 4

Preparation of 2-Chloro-4-(2′,2′-dimethylpropionamido)-N-cyclopropyl--N-methylbenzamide.

4-Amino-2-chloro-N-cyclopropyl-N-methylbenzamide (1.0g) from the previous reaction and triethylamine (0.55g) were stirred in methylene chloride (20ml) at 0-5°C. 2,2-Dimethylpropionyl chloride (0.60g) was then added dropwise over 5 minutes keeping the temperature below 4°C, the reaction stirred at that temperature for 20 minutes and then warmed to room temperature. The mixture was then extracted with methylene chloride and the organic layer washed with dilute aqueous hydrochloric acid, and then aqueous sodium bicarbonate, and dried over magnesium sulphate. Evaporation of the organic solvent gave the desired product as an off-white solid (1.38g), which was recrystallised to give pure compound (0.86g), mp 148-149°C; $^1H$ NMR ($CDCl_3$, 270MHz) δ: 0.50(4H,d), 1.32(9H,s), 2.70(1H, quintet), 3.10(3H,s), 7.19 (1H,d), 7.39 (1H,dd), 7.72 (1H,d) ppm.

The following are examples of compositions suitable for agricultural and horticultural purposes which can be formulated from the compounds of the invention. Such compositions form another aspect of the invention Percentages are by weight.

EXAMPLE 2

An emulsifiable concentrate is made up by mixing and stirring the ingredients until all are dissolved.

| | |
|---|---|
| Compound No. 5 of Table I | 10% |
| Benzyl alcohol | 30% |
| Calcium dodecylbenzenesulphonate | 5% |
| Nonylphenolethoxylate (13 mole ethylene oxide) | 10% |
| Alkyl benzenes | 45% |

EXAMPLE 3

The active ingredient is dissolved in methylene dichloride and the resultant liquid sprayed on to the granules of attapulgite clay. The solvent is then allowed to evaporate to produce a granular composition.

| | |
|---|---|
| Compound No. 5 of Table I | 5% |
| Attapulgite granules | 95% |

EXAMPLE 4

A composition suitable for use as a seed dressing is prepared by grinding and mixing the three ingredients.

| | |
|---|---|
| Compound No. 5 of Table I | 50% |
| Mineral oil | 2% |
| China clay | 48% |

EXAMPLE 5

A dustable powder is prepared by grinding and mixing the active ingredient with talc.

| | |
|---|---|
| Compound No. 5 of Table I | 5% |
| Talc | 95% |

EXAMPLE 6

A suspension concentrate is prepared by ball milling the ingredients to form an aqueous suspension of the ground mixture with water.

| | |
|---|---|
| Compound No. 5 of Table 1 | 40% |
| Sodium lignosulphonate | 10% |
| Bentonite clay | 1% |
| Water | 49% |

This formulation can be used as a spray by diluting into water or applied directly to seed.

EXAMPLE 7

A wettable powder formulation is made by mixing together and grinding the ingredients until all are thoroughly mixed.

| | |
|---|---|
| Compound No. 5 of Table I | 25% |
| Sodium lauryl sulphate | 2% |
| Sodium lignosulphonate | 5% |
| Silica | 25% |
| China clay | 43% |

EXAMPLE 8

The compounds were tested against a variety of foliar fungal diseases of plants. The technique employed was as follows.

The plants were grown in John Innes Potting Compost (No. 1 or 2) in 4cm diameter minipots. The test compounds were formulated either by bead milling with aqueous Dispersol T or as a solution in acetone or acetone/ethanol which was diluted to the required concentration immediately before use. For the foliage diseases, the formulations (100 ppm active ingredient) were sprayed onto the foliage and applied to the roots of the plants in the soil. The sprays were applied to maximum retention and the root drenches to a final concentration equivalent to approximately 40 ppm a.i. in dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading:

4 = no disease
3 = trace-5% of disease on untreated plants
2 = 6-25% of disease on untreated plants
1 = 26-59% of disease on untreated plants
0 = 60-100% of disease untreated plants

The results are shown in Table II.

TABLE II

| Compound No | Puccinia recondita (Wheat) | Erysiphe graminis tritici (Wheat) | Septoria nodorum (Wheat) | Venturia inaequalis (Apple) | Pyricularia oryzae (Rice) | Plasmopara viticola (Vine) | Phytophthora infestans (Tomato) |
|---|---|---|---|---|---|---|---|
| 1 | 0 | 0 | 0 | 3 | 0 | 4 | 2 |
| 2 | 0 | 0 | 0 | 4 | 0 | 4 | 0 |
| 3 | - | 0 | 0 | 0 | 0 | 4 | 3 |
| 5 | 0 | 0 | 0 | 4 | 0 | 4 | 2 |
| 6 | - | 0 | 0 | 0 | 0 | 0 | 4 |

CHEMICAL FORMULAE

(in description)

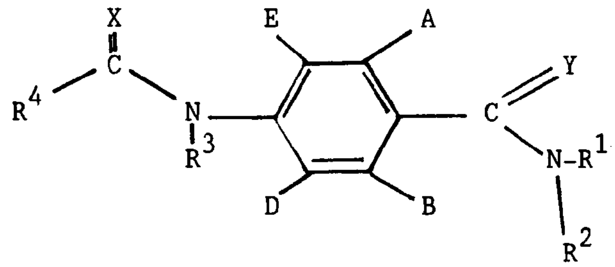

(I)

Scheme 1

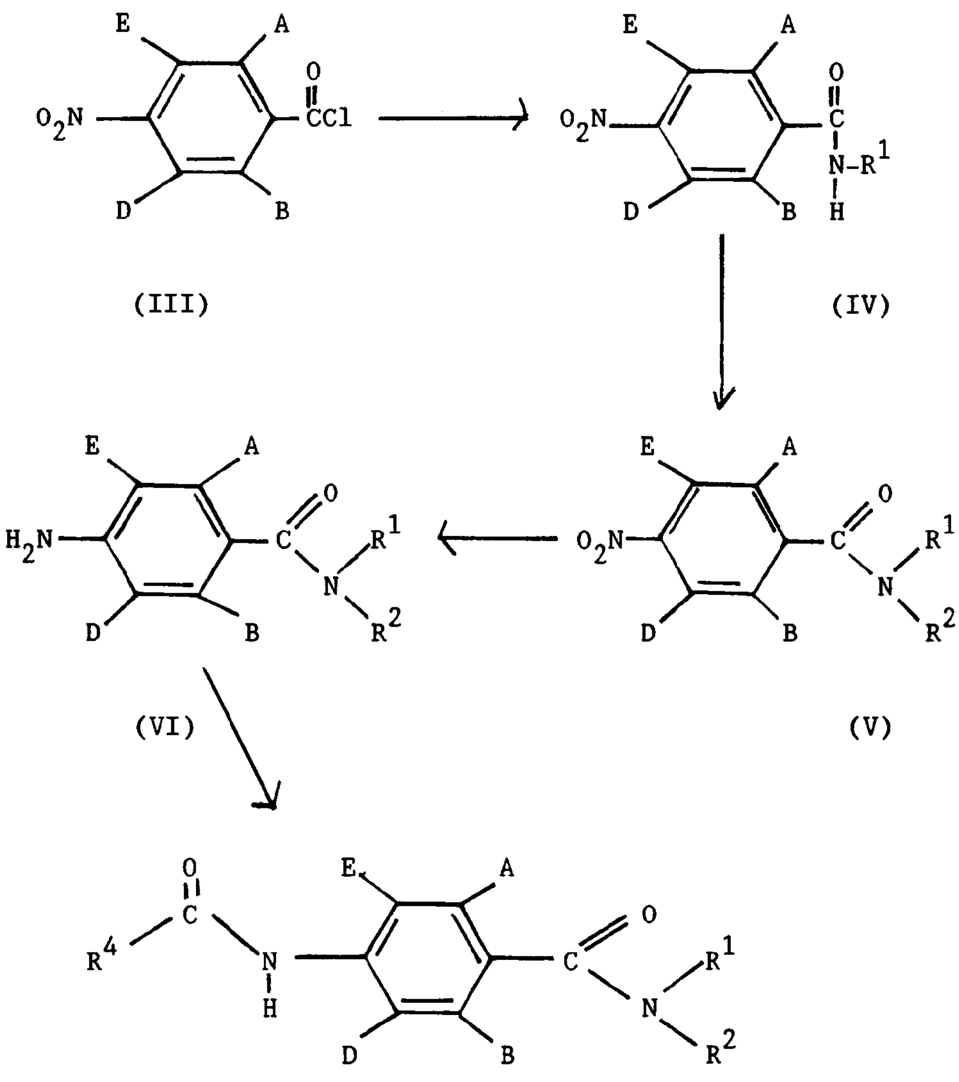

(III)

(IV)

(V)

(VI)

(II)

Scheme 2

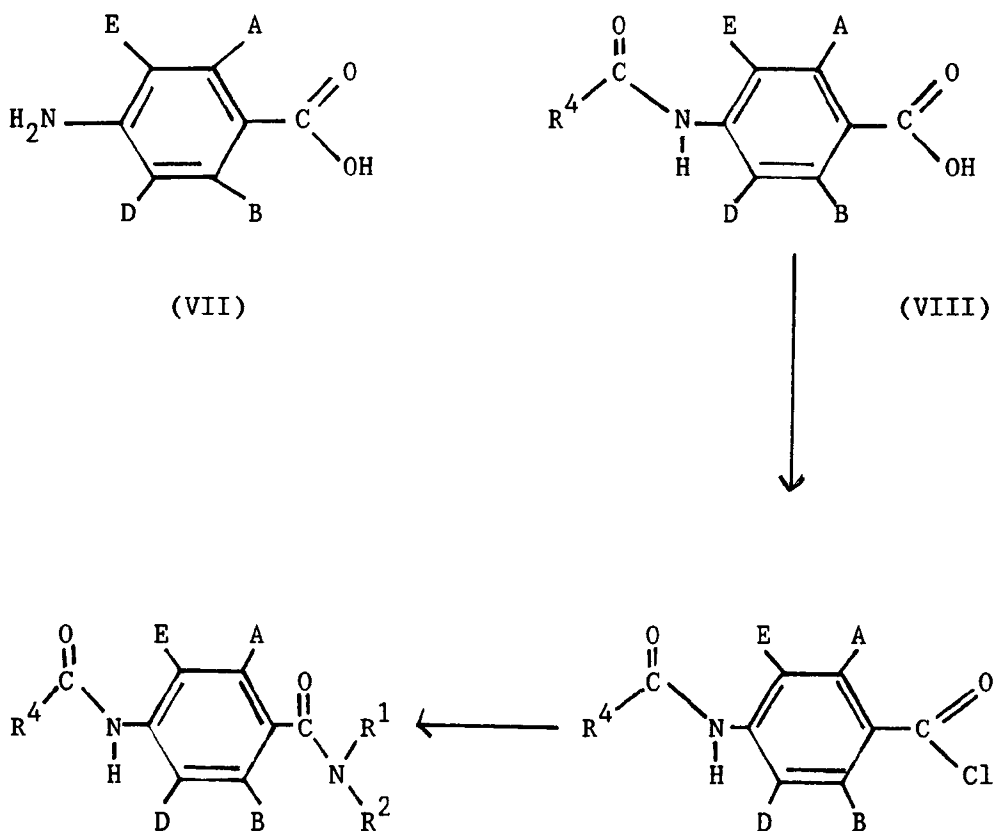

(VII) (VIII)

(II) (IX)

TABLE 1

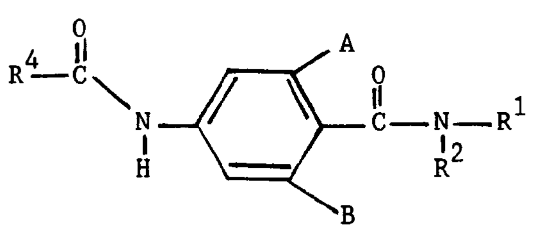

(I.1)

## Claims

1. A compound of the formula (I):

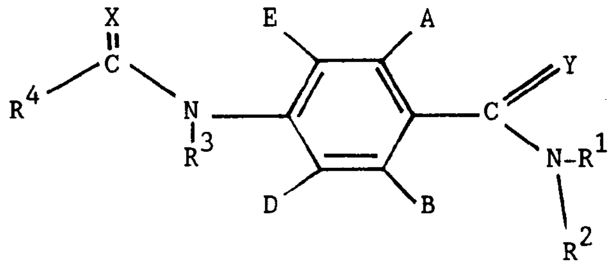

(I)

in which A and B are independently H, fluoro, chloro, bromo, iodo, $C_{1-4}$ alkyl, $C_{1-4}$ alkoxy, halo($C_{1-4}$)alkyl, cyano, $C_{1-4}$ alkoxycarbonyl, $C_{1-4}$ alkoxymethyl, $C_{1-4}$ alkylthiomethyl, $C_{2-4}$ alkenyl, $C_{2-4}$ alkynyl, formyl, $C_{1-4}$ alkylthio, carboxy, $C_{1-4}$ alkylcarbonyl or nitro, provided that both A and B are not H; D and E are independently H or fluoro; $R^1$ is $C_{2-4}$ alkenyl (optionally substituted with halogen), $C_{2-4}$ alkynyl (optionally substituted with halogen), halo($C_{1-4}$)alkyl, $C_{3-6}$ cycloalkyl, $C_{3-6}$ cycloalkyl($C_{1-4}$)alkyl, $C_{1-4}$ alkoxy($C_{1-4}$)alkyl, $C_{1-4}$ alkylthio($C_{1-4}$)alkyl or cyano; $R^2$ is $C_{1-4}$ alkyl, $C_{1-4}$ alkoxy, $C_{2-4}$ alkenyl (optionally substituted with halogen), $C_{2-4}$ alkynyl (optionally substituted with halogen), halo($C_{1-4}$)alkyl, $C_{3-6}$ cycloalkyl, $C_{3-6}$ cycloalkyl($C_{1-4}$)alkyl, $C_{1-4}$ alkoxy($C_{1-4}$)alkyl, $C_{1-4}$ alkylthio($C_{1-4}$)alkyl or cyano; $R^3$ is H; $R^4$ is $C_{2-8}$ alkyl, $C_{2-8}$ alkenyl, $C_{2-8}$ alkynyl or cyclopropyl (all of which are optionally substituted with halogen, $C_{1-4}$ alkyl, $C_{1-4}$ alkoxy or $C_{1-4}$ alkylthio), or $R^3$ and $R^4$, together with the group C(O)N to which they are attached, join to form an azetidin-2-one ring which is optionally substituted with $C_{1-4}$ alkyl; and X and Y are independently oxygen or sulphur.

2. A compound according to claim in which A is chloro or bromo; B and $R^3$ are both H; X and Y are both 0; $R^1$ is $C_{2-4}$ alkenyl, $C_{2-4}$ alkynyl, halo($C_{1-4}$)alkyl, cyclopropyl, $C_{1-4}$alkoxy($C_{1-4}$)alkyl, or $C_{1-4}$ alkylthio($C_{1-4}$-)alkyl; and $R^2$ is methyl.

3. A compound according to claim 1 or 2 in which $R^4$ is $R(CH_3)_2C$- where R is halogen, $C_{1-4}$ alkyl or $C_{1-4}$ alkoxy.

4. A compound according to claim 1 or 2 in which $R^4$ is $F(CH_3)_2C$-.

5. A compound according to claim 1 in which A is halo, methyl, $CF_3$, ethenyl or ethynyl; B is H or fluoro; E and D are both H; $R^1$ is allyl, 2-chloro- or 2-bromoalkyl, propargyl, 2-monofluoro- or 2-trifluoroethyl, cyclopropyl, methylthiomethyl, methoxyethyl or cyclopropylmethyl; $R^2$ is methyl, ethyl, allyl or propargyl; $R^3$ is H; and $R^4$ is $(CH_3)_3C$, $F((CH_3)_2C$, $CH_3CH_2(CH_3)_2C$, $F(CH_3CH_2)(CH_3)C$, $F((CH_3)_2C)(CH_3)C$, $F(CH_3CH_2)(CH_3CH_2)C$ or $F(FCH_2)(CH_3)C$.

6. A process for preparing a compound according to claim 1, in which X and Y are both oxygen and $R^3$ is hydrogen, which comprises reacting in a suitable organic solvent in the presence of a base either

(a) a compound of formula (VI):

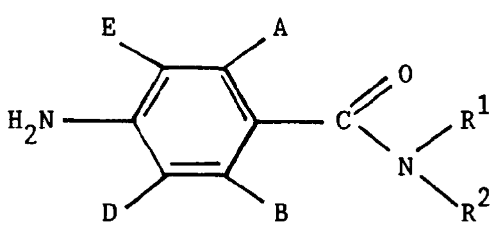

(VI)

with an acid chloride $R^4COCl$; or

(b) a compound of formula (IX):

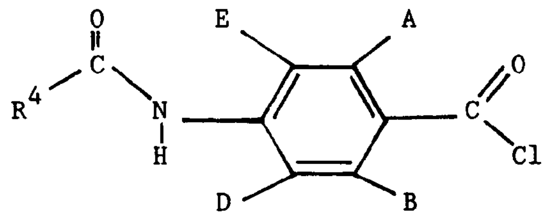

(IX)

with an amine $R^1R^2NH$; wherein A, B, D, E, $R^1$, $R^2$ and $R^4$ have the meanings given in claim 1.

**7.** A fungicidal composition comprising a fungicidally effective amount of a compound according to claim 1 and a fungicidally acceptable carrier or diluent therefor.

**8.** A method of combating fungi which comprises applying to plants, to the seeds of plants or to the locus of the plants or seeds, a compound according to claim 1 or a composition according to claim 7.

European Patent Office

# EUROPEAN SEARCH REPORT

Application Number

EP 91 30 6445

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D, P | EP-A-381330 (IMPERIAL CHEMICAL INDUSTRIES PLC)<br>* the whole document *<br>--- | 1-8 | C07C237/42<br>C07D205/08<br>C07D295/18<br>C07C327/38<br>C07C271/28<br>C07C275/42<br>C07C323/52<br>C07C317/44<br>A01N37/46<br>A01N53/00<br>A01N43/44<br>A01N47/20<br>A01N47/30 |
| A | FR-A-2369263 (ANPHAR S.A.)<br>* page 2, lines 19 - 39; claim 1 *<br>--- | 1 | |
| A | CHEMICAL ABSTRACTS, vol. 92, no. 3, 21 January 1980<br>Columbus, Ohio, USA<br>HASSAN K. M. ET AL.: "STUDIES ON LACTAMS AND THIAZOLIDINONES."<br>& INDIAN J. CHEM. SECT. B 1979 18B[1] 44-6<br>page 682; left-hand column; ref. no. 22465G<br>* abstract *<br>--- | 1 | |
| A<br><br>D | EP-A-2309 (IMPERIAL CHEMICAL INDUSTRIES LIMITED)<br>* the whole document *<br>& US-A-4282218<br>--- | 1 | |
| A,D | EP-A-127990 (SUMITOMO CHEMICAL COMPANY LIMITED)<br>* pages 13 - 21; claims 1, 3, 4 *<br>--- | 1, 7 | TECHNICAL FIELDS SEARCHED (Int. Cl.5 ) |
| A | PATENT ABSTRACTS OF JAPAN<br>vol. 6, no. 40 (C-94)(918) 12 March 1982,<br>& JP-A-56 156246 (HODOGAYA KAGAKU KOGYO K.K.) 02 December 1981,<br>* the whole document *<br>----- | 1 | C07C<br>C07D<br>A01N |

The present search report has been drawn up for all claims

| Place of search | Date of completion of the search | Examiner |
|---|---|---|
| BERLIN | 14 OCTOBER 1991 | RUFET, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)